# EUROPEAN PATENT APPLICATION

(11) **EP 3 202 443 A1**
(43) Date of publication of application: **09.08.2017**
(21) Application number: 16153980.4
(22) Date of filing: 02.02.2016
(51) Int. Cl.: A61M 5/24, A61M 5/315

(54) **PISTON WASHER FOR A DRUG DELIVERY DEVICE, METHOD OF PREPARING SUCH PISTON WASHER AND METHOD OF ASSEMBLY OF A DRUG DELIVERY DEVICE**

(71) Applicant: NOVO NORDISK A/S, 2880 Bagsværd (DK)
(72) Inventor: BENGTSSON, Henrik, DK-2630 Taastrup (DK); FREDERIKSEN, Lasse, DK-2665 Vallensbæk Strand (DK); PREUTHUN, Jan Harald, DK-2700 Brønshøj (DK)

(57) **Abstract**

Described is a method of preparing a piston washer (100) for use in a drug delivery device and for transferring a distally directed axial force from a piston rod towards a piston of a held cartridge. The method comprises the steps of: a) providing a piston washer (100) comprising a deformable portion made from a plastically deformable material, the piston washer defining a proximal surface geometry (114) configured for engagement with the distal portion of a piston rod and a distal surface geometry (126) configured for engagement with the proximal surface of a piston of a cartridge wherein the proximal surface geometry (114) and the distal surface geometry (126) are arranged axially relative to each other with an initial axial spacing, b) arranging the piston washer in a tool arrangement (300), and c) using the tool arrangement (300) to apply plastical deformation of the deformable portion of the piston washer (100) thereby modifying the axial spacing between the proximal surface geometry and the distal surface geometry until a target axial spacing is obtained.

## Description

The present invention relates to an assembly of components for a drug delivery device that allows a user to select single or multiple doses of an injectable liquid drug and to dispense the set dose of the product and to apply said product to a patient, preferably by injection. In particular, the present invention relates to a piston washer and a method of preparing such piston washer.

### BACKGROUND

In the disclosure of the present invention reference is mostly made to drug delivery devices used e.g. in the treatment of diabetes by delivery of insulin, however, this is only an exemplary use of the present invention.

Drug delivery devices allowing for multiple dosing of a required dosage of a liquid medicinal product, such as liquid drugs, and further providing administration of the liquid to a patient, are as such well-known in the art. Generally, such devices have substantially the same purpose as that of an ordinary syringe. Drug delivery devices of this kind have to meet a number of user specific requirements. For instance in case of those with diabetes, many users will be physically infirm and may also have impaired vision. Therefore, these devices need to be robust in construction, yet easy to use, both in terms of the manipulation of the parts and understanding by a user of its operation. Further, the dose setting must be easy and unambiguous and where the device is to be disposable rather than reusable, the device should be inexpensive to manufacture and easy to dispose. In order to meet these requirements, the number of parts and steps required to assemble the device and an overall number of material types the device is made from have to be kept to a minimum.

Typically, the liquid drug to be administered is provided in a cartridge that has a moveable piston or bung mechanically interacting with a piston rod of an expelling mechanism of the drug delivery device. By applying thrust to the piston in distal direction, a predefined amount of the liquid drug is expelled from the cartridge. Due to inevitable manufacturing tolerances there may for instance persist axial clearance between a cartridge's piston and the piston rod. Typically, prior to a primary use of the device, an end-user has to conduct a so-called priming of the expelling mechanism in order to ensure, that already with an initial dose setting and a first subsequent dose dispensing step, an accurate amount of the liquid drug is dispensed in a predefined way. An initial dose setting and expelling of a minor dose may in certain situations also be required for removing any air present in the cartridge and/or a connected needle and for performing a flow check.

Document WO 99/38554 A1 discloses several embodiments of injection devices each suitable for forming a disposable device wherein a liquid drug cartridge is inserted into the device during assembly in a production line. State of the art pen-type drug delivery devices that incorporate a dose setting feature often include a so-called end-of-content limiter to prevent a user from selecting a size of a dose which exceeds the amount of liquid drug remaining in a cartridge of the device. References WO 01/19434 A1, WO 2006/128794 A2 and WO 2010/149209 A1 include disclosure of such end-of-content limiters.

In the production line, during final assembly operations of the devices, at least a part of the priming is typically carried out using the dose setting and expelling mechanism so that users will experience virtually consistent requirement for a priming operation across individual pen samples irrespective of the initial gap between the piston rod and the piston which emanates from tolerances. References WO 2009/095332 A1 disclose devices wherein a distance between the distal end of a piston rod means and the piston is minimized or reduced to zero. However, the solutions are not readily suitable for use where a piston rod is rotated during dose expelling. Further, for eliminating clearance between piston and piston rod, reference WO 2011/051365 A2 suggests to select and insert at least one spacer from a set of differently sized spacers. However, in high volume production, the use of a large number of differently sized spacers with very small steps in dimensions introduces a risk of spacers of different sizes being mixed up. WO2011/039226 A1 further suggests a method for adjusting the effective length of a piston rod by gradually deforming a part of the piston rod during the assembly process of a drug delivery device. As the amount of deformation results in varying degrees of compression of the bung of the cartridge during deformation the process may be difficult to control. in addition, non-symmetric deformation may easily occur.

It is an object of the present invention to provide a piston washer for a drug delivery device featuring improved and facilitated clearance reduction or clearance elimination. It is a further object of the invention to provide a simplified and robust method of eliminating clearance in a drug delivery device. Finally, it is an object of the invention to provide manufacture of a piston washer and drug delivery devices incorporating such piston washer providing consistently uniform and predictable total doseable amount of liquid drug from a held cartridge.

In the disclosure of the present invention, embodiments and aspects will be described which will address one or more of the above objects or which will address objects apparent from the below disclosure as well as from the description of exemplary embodiments.

### SUMMARY

In a first aspect, the present invention relates to a method of preparing a piston washer for use in a drug delivery device for transferring a distally directed axial force from a piston rod towards a piston of a held cartridge. The method may comprise the steps of:
a) providing a piston washer comprising a deformable portion made from a plastically deformable material, the piston washer defining a proximal surface geometry configured for engagement with the distal portion of a piston rod and a distal surface geometry configured for engagement with the proximal surface of a piston of a cartridge wherein the proximal surface geometry and the distal surface geometry are arranged axially relative to each other with an initial axial spacing,
b) arranging the piston washer in a tool arrangement,
c) using the tool arrangement to apply plastical deformation of the deformable portion of the piston washer thereby modifying the axial spacing between the proximal surface geometry and the distal surface geometry until a target axial spacing is obtained.

By using plastical deformation of the piston washer in a tool-arrangement for modifying an initial axial spacing of the piston washer the preparation of the washer may occur as part of in-line production enabling use of fewer variants of components that makes up each sample of the drug delivery devices. At the same time a superior control of process parameters is obtained leaving no potential impact on the components of the drug delivery device intended to receive the piston washer. In particular for applications where a slight precompression of the piston of a held cartridge is aimed at, a consistent precompression of the piston of the cartridge may be obtained where the precompression is near-constant across the different device samples irrespective of the magnitude of tolerance variances of the involved components.

Further, in applications of drug delivery devices where the piston rod is configured for rotation relative to the piston washer during dose expelling, the tool-arrangement may be so configured that deformation of the piston washer is provided while ensuring precise rotation symmetric deformation. This enables superior conditions for rotation of the piston rod during dose expelling.

The piston washer may in some embodiments define a deformable portion wherein the deformable portion only forms a fraction of the entire piston washer. In other embodiments, the entire piston washer is as such deformable and the piston washer thus forms said deformable portion.

In the context of an embodiment of the present invention, the tool arrangement is defined as a tool arrangement which is arranged for receiving the piston washer as a separate component and thus configured so that the piston washer is plastically deformed in the tool arrangement while being maintained separated from the piston rod and separated from the piston of a cartridge.

In certain embodiments the piston washer defines a peripheral section and a central section wherein the peripheral section comprises a distal peripheral surface and a proximal peripheral surface and wherein the central section defines a distal central surface and a proximal central surface, and wherein the proximal central surface includes a recessed central area configured for receiving the distal portion of the piston rod.

In certain embodiments the recessed central area is so formed that it acts as a bearing surface for engagement with the distal end of the piston rod allowing low-friction rotational movement of the piston rod relative to the proximal part of the piston washer.

The extreme distal surface of the piston washer may in some embodiments be defined by the distal surface of the peripheral section. The distal peripheral section may be formed to exhibit a diameter slightly smaller than the piston of the cartridge. This ensures a large contact area between the piston washer and the piston of the cartridge.

A bridge section may be provided for interconnecting the central section and the peripheral section wherein the bridge section at least partly defines said deformable portion.

In the method step c) of applying plastical deformation the step may comprise applying controlled pressure on the distal central surface and/or the proximal central surface for axially moving the central section of the piston washer relative to the peripheral section.

In some embodiments the central section is deformed in the proximal direction away from the distal surface geometry. In other embodiments, the central section is deformed in the distal direction towards the distal surface geometry.

The tool arrangement may in some embodiments comprise a first peripheral tool portion configured for engaging the distal peripheral surface of the piston washer and a second peripheral tool portion configured for engaging the proximal peripheral surface of the piston washer. The method step b) of arranging the piston washer in a tool arrangement may in some embodiments comprise the step of axially clamping the peripheral section of the piston washer between the first peripheral tool portion and the second peripheral tool portion. The axial clamping is maintained during the deformation provided in step c). In this way a controlled deformation of central portions of the piston washer may be provided while leaving the peripheral section of the piston substantially non-deformed.

A first central tool portion may be arranged for engaging the distal central surface of the piston washer and a second central tool portion may be arranged for engaging the proximal central surface of the piston washer. In the method step of applying plastical deformation is provided by moving the first central tool portion and/or the second central tool portion axially relative to the first and second peripheral tool portion.

In some embodiments, the first central tool portion and the second central tool portion axially engages the central section of the piston washer at different sides thereof. The first central tool portion and the second central tool portion may be moved axially in the same direction relative to both the first peripheral tool portion and the second peripheral tool portion to reduce or increase the axial spacing between the proximal surface geometry and the distal surface geometry.

In some embodiments, the first central tool portion and the second central tool portion are moved axially in synchronism relative to the first peripheral tool portion and the second peripheral tool portion to reduce or increase the axial spacing between the proximal surface geometry and the distal surface geometry.

The deformable portion of the piston washer is heated prior to or during plastical deformation of the deformable portion.

In a second aspect, the invention relates to a piston washer for use in a drug delivery device for transferring a distally directed axial force from a piston rod towards a piston of a held cartridge, the piston washer comprising:
- a proximal surface geometry configured for engagement with the distal portion of a piston rod, and
- a distal surface geometry configured to engage and abut a piston of a held cartridge,
wherein the piston washer comprises a deformable portion made from a plastically deformable material and wherein the proximal surface geometry and the distal surface geometry are arranged axially relative to each other with a target axial spacing obtained by deformation of the deformable portion.

In a third aspect, the invention relates to a method of assembling a drug delivery device comprising a piston washer as prepared in accordance with any of the variants of the first aspect. The method comprises the steps of:
- providing a cartridge comprising a liquid drug and a piston slideably arranged therein in a first housing component to form a cartridge sub-assembly,
- providing a dose setting and expelling mechanism comprising the piston rod in a second housing component to form a housing sub-assembly,
- determining an axial position of a proximal end face of the piston of a cartridge of the cartridge sub-assembly,
- determining an axial position of a distal end face of a piston rod of a drive mechanism pre-assembled in a second housing component,
- determining or estimating the size of axial clearance between the piston and the piston rod if the first housing component and the second housing component were assembled,
- providing the piston washer prepared in accordance with the first aspect, wherein the target axial spacing corresponds to or correlates with said axial clearance, and
- mutually interconnecting the first and the second housing components with the piston washer positioned between the piston rod and the piston.

In a fourth aspect, the invention relates to a drug delivery device for expelling a dose of drug from a held cartridge, the drug delivery device defining a distal drug outlet end and an opposite proximal end and comprising:
- a housing component holding a cartridge comprising a liquid drug and a piston slideable arranged therein in an axial direction,
- a dose setting and expelling mechanism coupled to the housing component and comprising a piston rod for exerting a force on the piston of the cartridge in a distal direction for expelling a dose, and
wherein a piston washer prepared in accordance with the first aspect is arranged axially between the piston of the cartridge and the piston rod.

The dose setting and expelling mechanism of the drug delivery device may be so configured that the piston rod rotates during dose expelling. The piston rod may be rotatably arranged relative to the piston washer to allow the piston rod to rotate while the piston washer remains non-rotatable.

The piston washer may be configured for being loosely withheld between the piston rod of the drug delivery device and the piston of a held cartridge. Hence, for some embodiments, when the piston washer assumes its final configuration in a drug delivery device, the piston rod will be able to rotate independently from the piston washer, i.e. allowing the piston washer to be kept non-rotatable while the piston rod rotates, e.g. during dose expelling. In other embodiments, the piston washer includes a geometry which axially couples to the piston rod so that the piston washer and the piston rod are axially locked relative to each other while allowing the piston rod to rotate relative to the piston washer. In still other embodiments, the piston washer maintains its rotational position relative to the piston rod during dose expelling.

The piston rod of the drug delivery device may be configured for being rotated during dose expelling such as when a thread of the piston rod engages with a threaded nut arranged at a fixed position within the housing. In such embodiment, the piston rod may be rotatably arranged relative to the piston washer to thereby allow the piston rod to rotate while the piston washer remains non-rotatable during dose expelling.

The dose setting and expelling mechanism may include a driver that serves to move the piston rod axially in the distal direction during dose expelling.

The driver may in some embodiments be so configured that during dose expelling the driver induces rotation of the piston rod which is threadedly engaged with a nut that is kept stationary during dose expelling. The dose setting and expelling mechanism may further comprise a dose setting element that is configured to rotate relative to the driver during setting of a dose and wherein the dose setting element does not rotate relative to the driver during expelling of a set dose so that these components rotate together during dose expelling.

In a further embodiment of the invention an end-of-content limiter may be arranged between the driver and the dose setting element of the drug delivery device. The end-of-content limiter is configured to prevent setting of a dose which exceeds a doseable amount of liquid drug remaining in the cartridge. By using a piston washer in accordance with the different aspects of the invention, the function of the end-of-content limiter will not be operated or influenced by tolerance elimination during manufacture and assembly of the device.

In exemplary embodiments the end-of-content limiter is engaging the driver and the dose setting element. The end-of-content limiter moves towards an end-of-content stop geometry for example provided by one of the driver and the dose setting element as the dose setting element is rotated relative to the driver for dialling up a dose.

As used herein, the term "insulin" is meant to encompass any drug-containing flowable medicine capable of being passed through a delivery means such as a cannula or hollow needle in a controlled manner, such as a liquid, solution, gel or fine suspension, and which has a blood glucose controlling effect, e.g. human insulin and analogues thereof as well as non-insulins such as GLP-1 and analogues thereof. In the description of exemplary embodiments reference will be made to the use of insulin.

### BRIEF DESCRIPTION OF DRAWINGS

In the following the invention will be further described with reference to the drawings, wherein
fig. 1 shows a perspective view of a prior art pen device,
fig. 2 shows in an exploded view the components of the pen device of fig. 1,
figs. 3A and 3B show in sectional views an expelling mechanism of the pen device of fig. 1 in two states,
figs. 3C - 3E show components of the pen device of fig. 1,
figs. 4 and 5 schematically illustrate the effect of tolerance variations for piston rod position and piston position for four sample pen devices during device assembly,
fig. 6a shows a perspective view of the distal surface of a first embodiment of a piston washer according to the invention,
fig. 6b shows a cross-sectional plan view of the piston washer shown in fig. 6a, fig. 6c shows a perspective view of proximal surface of the piston washer shown in fig. 6a,
fig. 7 schematically illustrates an adjustment procedure for adjusting the piston washer shown in fig. 6a,
fig. 8a shows an example piston washer according to the invention in an initial state defining an initial axial spacing geometry,
fig. 8b shows the piston washer of fig. 8a after the piston washer has been permanently deformed into a target axial spacing geometry, and
fig. 9 schematically illustrates sample components for four different pen samples each including a piston washer of fig. 6a adjusted for a respective target axial spacing to compensate for tolerance variations.

Corresponding reference characters indicate corresponding parts throughout the several views. Although the drawings represent embodiments of the present invention, the drawings are not necessarily to scale, and certain features may be exaggerated or omitted in some of the drawings in order to better illustrate and explain the present invention.

### DESCRIPTION

When in the following terms such as "upper" and "lower", "right" and "left", "horizontal" and "vertical" or similar relative expressions are used, these only refer to the appended figures and not necessarily to an actual situation of use. The shown figures are schematic representations for which reason the configuration of the different structures as well as their relative dimensions are intended to serve illustrative purposes only. When the term member or element is used for a given component it generally indicates that in the described embodiment the component is a unitary component, however, the same member or element may alternatively comprise a number of sub-components just as two or more of the described components could be provided as unitary components, e.g. manufactured as a single injection moulded part. The term "assembly" does not imply that the described components necessarily can be assembled to provide a unitary or functional assembly during a given assembly procedure but is merely used to describe components grouped together as being functionally more closely related.

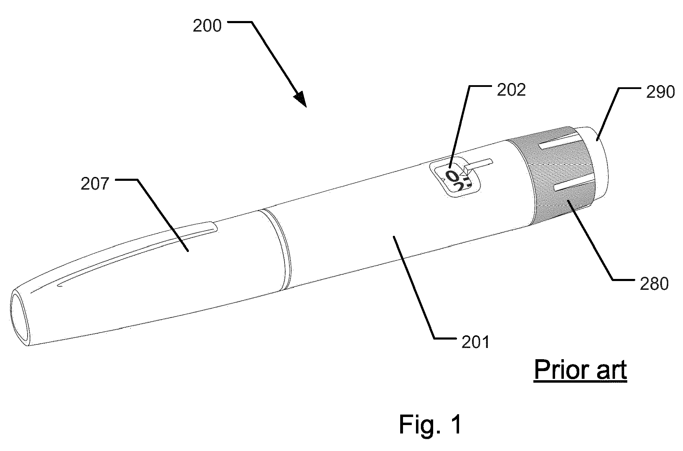

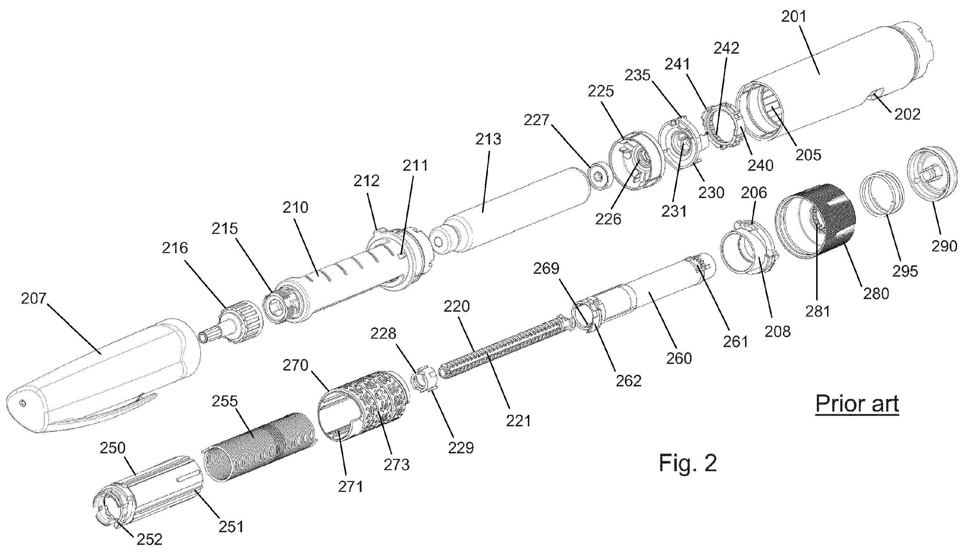

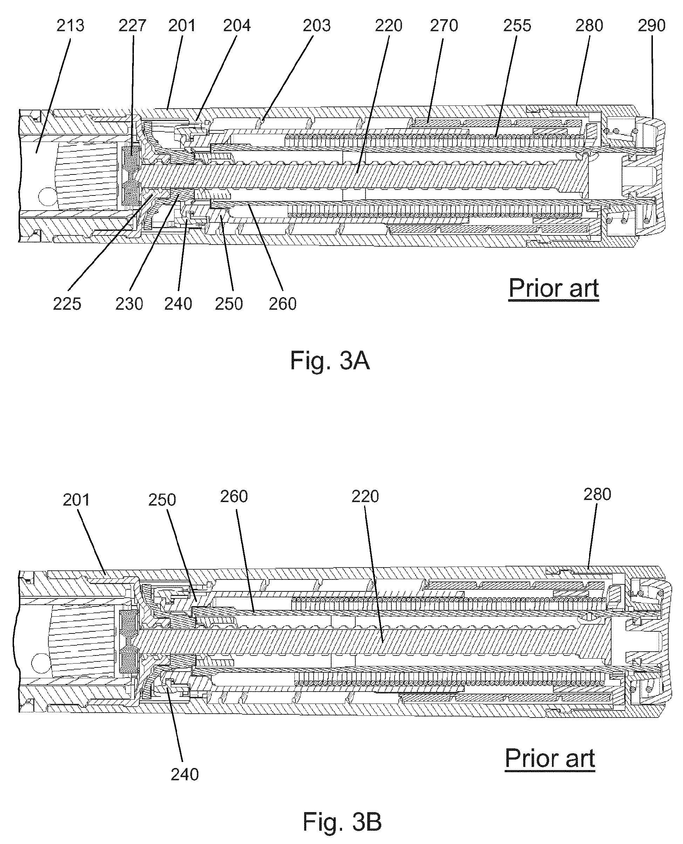

Figs. 1 shows a prior art drug delivery device in the form of a pen-formed auto-injection device 200, i.e. a so-called "injection pen" that includes an expelling mechanism incorporating a spring drive. Fig. 2 shows an exploded view of the prior art auto-injection device 200 shown in fig. 1. Figs. 3A and 3B show cross sectional views of the expelling mechanism of the prior art auto-injection device 200 shown in figs. 1 and 2 where fig. 3A shows the device in dose setting state and fig. 3B shows the device in dose expelling state.

In the present context the device 200 represents a "generic" drug delivery device providing a specific example of a device which, in accordance with the present invention, may be modified in order to obtain a device that provides improved user feedback. As the invention relates to elements of a device which mainly pertains to user feedback, an exemplary embodiment of such a device will be described for better understanding of the invention.

The pen device 200 comprises a cap part 207 and a main part having a proximal body or drive assembly portion with a housing 201 in which a drug expelling mechanism is arranged or integrated, and a distal cartridge holder portion in which a drug-filled transparent cartridge 213 with a distal needle-penetrable septum is arranged and retained in place by a non-removable cartridge holder attached to the proximal portion, the cartridge holder having openings allowing a portion of the cartridge to be inspected as well as distal coupling means 215 allowing a needle assembly to be releasably mounted. The cartridge is provided with a piston driven by a piston rod forming part of the expelling mechanism and may for example contain an insulin, GLP-1 or growth hormone formulation. A proximal-most rotatable dose dial member 280 serves to manually set a desired dose of drug shown in display window 202 and which can then be expelled when the release button 290 is actuated. Depending on the type of expelling mechanism embodied in the drug delivery device, the expelling mechanism may comprise a spring as in the shown embodiment which is strained during dose setting and then released to drive the piston rod when the release button 290 is actuated.

As appears, fig. 1 shows a drug delivery device of the pre-filled type, i.e. it is supplied with a pre-mounted cartridge and is to be discarded when the cartridge has been emptied. In alternative embodiments, and in accordance with the present invention, the drug delivery device may be designed to allow a loaded cartridge to be replaced, e.g. in the form of a "rear-loaded" drug delivery device in which the cartridge holder is adapted to be removed from the device main portion, or alternatively in the form of a "front-loaded" device in which a cartridge is inserted through a distal opening in the cartridge holder which is non-removable attached to the main part of the device.

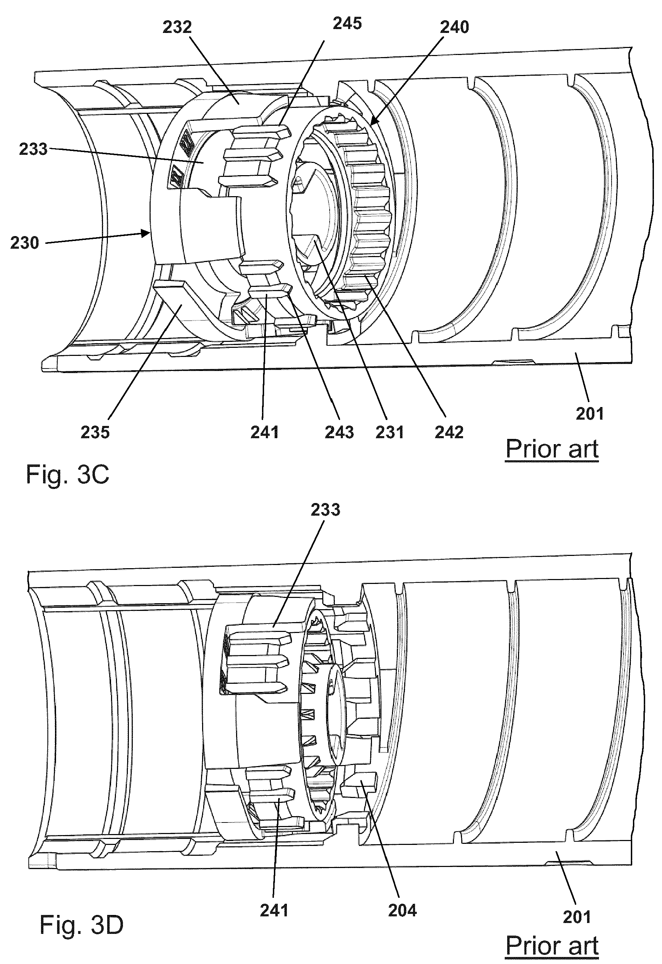

More specifically, referring to fig. 2, the pen comprises a tubular housing 201 with a window opening 202 and onto which a cartridge holder 210 is fixedly mounted, a drug-filled cartridge 213 being arranged in the cartridge holder. The cartridge holder is provided with distal coupling means 215 allowing a needle assembly 216 to be releasably mounted, proximal coupling means in the form of two opposed protrusions 211 allowing a cap 207 to be releasably mounted covering the cartridge holder and a mounted needle assembly, as well as a protrusion 212 preventing the pen from rolling on e.g. a table top. In the housing distal end a nut element 225 is fixedly mounted, the nut element comprising a central threaded bore 226, and in the housing proximal end a spring base member 208 with a central opening is fixedly mounted. A drive system comprises a threaded piston rod 220 having two opposed longitudinal grooves and being received in the nut element threaded bore, a ring-formed piston rod drive element 230 rotationally arranged in the housing, and a ring-formed clutch element 240 which is in rotational engagement with the drive element (see below), the engagement allowing axial movement of the clutch element. The clutch element is provided with outer spline elements 241 adapted to engage corresponding splines on the housing inner surface, this allowing the clutch element to be moved between a rotationally locked proximal position, in which the splines are in engagement, and a rotationally free distal position in which the splines are out of engagement. As just mentioned, in both positions the clutch element 240 is rotationally locked to the drive element 230. The drive element comprises a central bore with two opposed protrusions 231 in engagement with the grooves on the piston rod whereby rotation of the drive element results in rotation and thereby distal axial movement of the piston rod due to the threaded engagement between the piston rod and the nut element. The drive element further comprises a pair of opposed circumferentially extending flexible ratchet arms 235 adapted to engage corresponding ratchet teeth 205 arranged on the housing inner surface. The drive element and the clutch element comprise cooperating coupling structures rotationally locking them together but allowing the clutch element to be moved axially, this allowing the clutch element to be moved axially to its distal position in which it is allowed to rotate, thereby transmitting rotational movement from the dial system (see below) to the drive system. The interaction between the clutch element, the drive element and the housing will be shown and described in greater detail with reference to figs. 3C and 3D.

On the piston rod an end-of-content (EOC) member 228 (EOC limiter) is threadedly mounted and on the distal end a washer 227 is rotationally mounted. The EOC member comprises a pair of opposed radial projections 229 for engagement with the reset tube (see below).

The dial system comprises a ratchet tube 250, a reset tube 260, a scale drum 270 with an outer helically arranged row of dose numerals, a user-operated dose dial member 280 for setting a dose of drug to be expelled, a release button 290 and a torque spring 255 (see fig. 3A and 3B). The reset tube is mounted axially locked inside the ratchet tube but is allowed to rotate a few degrees (see below). The reset tube comprises on its inner surface two opposed longitudinal grooves 269 adapted to engage the radial projections 229 of the EOC member, whereby the EOC can be rotated by the reset tube but is allowed to move axially. The clutch element is mounted axially locked on the outer distal end portion of the ratchet tube 250, this providing that the ratchet tube can be moved axially in and out of rotational engagement with the housing via the clutch element. The dose dial member 280 is mounted axially locked but rotationally free on the housing proximal end, the dose dial member being under normal operation rotationally locked to the reset tube (see below), whereby rotation of dose dial member results in a corresponding rotation of the reset tube and thereby the ratchet tube. The release button 290 is axially locked to the reset tube but is free to rotate. A return spring 295 provides a proximally directed force on the button and the thereto mounted reset tube. The scale drum 270 is arranged in the circumferential space between the ratchet tube and the housing, the drum being rotationally locked to the ratchet tube via cooperating longitudinal splines 251, 271 and being in rotational threaded engagement with the inner surface of the housing via cooperating thread structures 203, 273, whereby the row of numerals passes the window opening 202 in the housing when the drum is rotated relative to the housing by the ratchet tube. The torque spring is arranged in the circumferential space between the ratchet tube and the reset tube and is at its proximal end secured to the spring base member 208 and at its distal end to the ratchet tube, whereby the spring is strained when the ratchet tube is rotated relative to the housing by rotation of the dial member. A ratchet mechanism with a flexible ratchet arm 252 is provided between the ratchet tube and the clutch element, the latter being provided with an inner circumferential teeth structures 242, each tooth providing a ratchet stop such that the ratchet tube is held in the position to which it is rotated by a user via the reset tube when a dose is set. In order to allow a set dose to be reduced a ratchet release mechanism 262 is provided on the reset tube and acting on the ratchet tube, this allowing a set dose to be reduced by one or more ratchet increments by turning the dial member in the opposite direction, the release mechanism being actuated when the reset tube is rotated the above-described few degrees relative to the ratchet tube.

Having described the different components of the expelling mechanism and their functional relationship, operation of the mechanism will be described next with reference mainly to figs. 3A and 3B.

The pen mechanism can be considered as two interacting systems, a dose system and a dial system, this as described above. During dose setting the dial mechanism rotates and a torsion spring of the spring drive is loaded. The dose mechanism is locked to the housing and cannot move. When the push button is pushed down, the dose mechanism is released from the housing and due to the engagement to the dial system, the torsion spring will now rotate back the dial system to the starting point and rotate the dose system along with it.

The central part of the dose mechanism is the piston rod 220, the actual displacement of the piston being performed by the piston rod. During dose delivery, the piston rod is rotated by the drive element 230 and due to the threaded interaction with the nut element 225 which is fixed to the housing, the piston rod moves forward in the distal direction. Between the rubber piston and the piston rod, the piston washer 227 is placed which serves as an axial bearing for the rotating piston rod and evens out the pressure on the rubber piston. As the piston rod has a non-circular cross section where the piston rod drive element engages with the piston rod, the drive element is locked rotationally to the piston rod, but free to move along the piston rod axis. Consequently, rotation of the drive element results in a linear forwards movement of the piston. The drive element is provided with small ratchet arms 234 which prevent the drive element from rotating clockwise (seen from the push button end). Due to the engagement with the drive element, the piston rod can thus only move forwards. During dose delivery, the drive element rotates anti-clockwise and the ratchet arms 235 provide the user with small clicks due to the engagement with the ratchet teeth 205, e.g. one click per unit of insulin expelled.

Turning to the dial system, the dose is set and reset by turning the dose dial member 280. When turning the dial, the reset tube 260, the EOC member 228, the ratchet tube 250 and the scale drum 270 all turn with it. As the ratchet tube is connected to the distal end of the torque spring 255, the spring is loaded. During dose setting, the arm 252 of the ratchet performs a dial click for each unit dialled due to the interaction with the inner teeth structure 242 of the clutch element. In the shown embodiment the clutch element is provided with 24 ratchet stops providing 24 clicks (increments) for a full 360 degrees rotation relative to the housing. The spring is preloaded during assembly which enables the mechanism to deliver both small and large doses within an acceptable speed interval. As the scale drum is rotationally engaged with the ratchet tube, but movable in the axial direction and the scale drum is in threaded engagement with the housing, the scale drum will move in a helical pattern when the dial system is turned, the number corresponding to the set dose being shown in the housing window 202.

The ratchet 252, 242 between the ratchet tube and the clutch element 240 prevents the spring from turning back the parts. During resetting, the reset tube moves the ratchet arm 252, thereby releasing the ratchet click by click, one click corresponding to one unit IU of insulin in the described embodiment. More specifically, when the dial member is turned clockwise, the reset tube simply rotates the ratchet tube allowing the arm of the ratchet to freely interact with the teeth structures 242 in the clutch element. When the dial member is turned counter-clockwise, the reset tube interacts directly with the ratchet click arm forcing the click arm towards the centre of the pen away from the teeth in the clutch, thus allowing the click arm on the ratchet to move "one click" backwards due to torque caused by the loaded spring.

To deliver a set dose, the release button 290 is pushed in the distal direction by the user as shown in fig. 3B. The reset tube 260 decouples from the dial member and subsequently the clutch element 240 disengages the housing splines 204. Now the dial mechanism returns to "zero" together with the drive element 230, this leading to a dose of drug being expelled. It is possible to stop and start a dose at any time by releasing or pushing the push button at any time during drug delivery. A dose of less than 5 IU normally cannot be paused, since the rubber piston is compressed very quickly leading to a compression of the rubber piston and subsequently delivery of insulin when the piston returns to the original dimensions.

The EOC feature prevents the user from setting a larger dose than left in the cartridge. The EOC member 228 is rotationally locked to the reset tube, which makes the EOC member rotate during dose setting, resetting and dose delivery, during which it can be moved axially back and forth following the thread of the piston rod. When it reaches the proximal end of the piston rod a stop is provided, this preventing all the connected parts, including the dial member, from being rotated further in the dose setting direction, i.e. the now set dose corresponds to the remaining drug content in the cartridge.

The scale drum 270 is provided with a distal stop surface adapted to engage a corresponding stop surface on the housing inner surface, this providing a maximum dose stop for the scale drum preventing all the connected parts, including the dial member, from being rotated further in the dose setting direction. In the shown embodiment the maximum dose is set to 80 IU. Correspondingly, the scale drum is provided with a proximal stop surface adapted to engage a corresponding stop surface on the spring base member, this preventing all the connected parts, including the dial member, from being rotated further in the dose expelling direction, thereby providing a "zero" stop for the entire expelling mechanism. In the following, the position that the dial member assumes after completion of the expelling of a set dose will be referred to as the "zero dose position".

To prevent accidental over-dosage in case something should fail in the dialing mechanism allowing the scale drum to move beyond its zero-position, the EOC member serves to provide a security system. More specifically, in an initial state with a full cartridge the EOC member is positioned in a distal-most axial position in contact with the drive element. After a given dose has been expelled the EOC member will again be positioned in contact with the drive element. Correspondingly, the EOC member will lock against the drive element in case the mechanism tries to deliver a dose beyond the zero-position. Due to tolerances and flexibility of the different parts of the mechanism the EOC will travel a short distance allowing a small "over dose" of drug to be expelled, e.g. 3-5 IU of insulin.

The expelling mechanism further comprises an end-of-dose (EOD) click feature providing a distinct feedback at the end of an expelled dose informing the user that the full amount of drug has been expelled. More specifically, the EOD function is made by the interaction between the spring base and the scale drum. When the scale drum returns to zero, a small click arm 206 on the spring base is forced backwards by the progressing scale drum. Just before "zero" the arm is released and the arm hits a countersunk surface on the scale drum.

The shown mechanism is further provided with a torque limiter in order to protect the mechanism from overload applied by the user via the dose dial member. This feature is provided by the interface between the dose dial member and the reset tube which as described above are rotationally locked to each other. More specifically, the dose dial member is provided with a circumferential inner teeth structure 281 engaging a number of corresponding teeth arranged on a flexible carrier portion 261 of the reset tube. The reset tube teeth are designed to transmit a torque of a given specified maximum size, e.g. 150-300 Nmm, above which the flexible carrier portion and the teeth will bend inwards and make the dose dial member turn without rotating the rest of the dial mechanism. Thus, the mechanism inside the pen cannot be stressed at a higher load than the torque limiter transmits through the teeth.

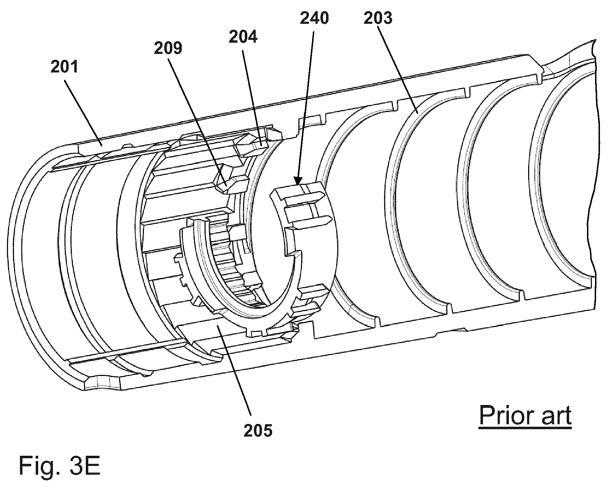

In fig. 3C the clutch element, the drive element and the housing (in partial) are shown in the dose setting state, and in fig. 3D the same components are shown in the expelling state. As appears, the piston rod on which the drive element is arranged and the ratchet tube on which the clutch element is mounted are not shown. To better show the structures provided on the inner surface of the housing fig. 3E shows a partial clutch element 240 arranged in the housing 201.

The inner surface of the housing 201 comprises a circumferential ring-formed array of axially oriented spline elements 204 protruding into the interior, each having a pointed distal end 209, as well as a circumferential ring-formed array of one-way ratchet teeth 205. The inner surface further comprises a male helical thread 203 adapted to engage the female helical thread 273 on the scale drum 270. A distal circumferential groove is formed to engage and mount the nut element 225. The clutch element 240 comprises an inner circumferential ring-formed array of ratchet teeth 242 adapted to engage the ratchet arm 252 on the ratchet tube 250, and an outer circumferential ring-formed array of axially oriented spline elements 241 adapted to engage the spline elements 204 of the housing as well as the coupling slots in the drive element (see below), each spline having a pointed proximal end 243. The drive element 230 comprises a pair of opposed coupling portions each comprising two proximally extending skirt portions 232 between which an axially extending coupling slot 233 is formed, the slot being adapted to engage a portion of the clutch element spline elements. In this way the engaging surfaces serve to transmit a rotational force and thereby torque from the clutch element to the drive element in the expelling state. The drive element further comprises a pair of opposed circumferentially extending flexible ratchet arms adapted to engage the ring-formed array of one-way ratchet teeth 205. During dose delivery, the drive element rotates anti-clockwise and the ratchet arms 235 also provide the user with small clicks due to the engagement with the ratchet teeth 205, e.g. one click per unit of insulin expelled. In the shown embodiment 24 ratchet teeth are provided corresponding to 15 degrees rotation per unit of insulin. The central bore of the drive element comprises two opposed protrusions 231 adapted to engage with the axially oriented grooves on the piston rod.

In the dose setting state shown in fig. 3C the spline elements 241 of the clutch element are in engagement with the spline elements 204 of the housing thereby rotationally locking the clutch element relative to the housing. As can be seen from fig. 3C a group of clutch spline elements are received in the corresponding coupling slot with a slight rotational play. In the expelling state shown in fig. 3D the spline elements 241 of the clutch element are moved distally out of engagement with the spline elements 204 of the housing thereby allowing rotation of the clutch element relative to the housing. As can be seen from fig. 3D the group of clutch spline elements are now received in the corresponding coupling slot without rotational play.

Fig. 3C shows the clutch element 240 showing the above-described inner circumferential ring-formed array of ratchet teeth 242 and the outer circumferential ring-formed array of axially oriented spline elements 241. As appears, the spline elements are not arranged equidistantly on the ring but in groups, the groups comprising two opposed coupling groups 245 serving as the coupling means engaging the coupling slots 233. Whereas thus only some of the spline elements serve as coupling means between the clutch element and the drive element they all serve as coupling means between the clutch element and the housing splines 204.

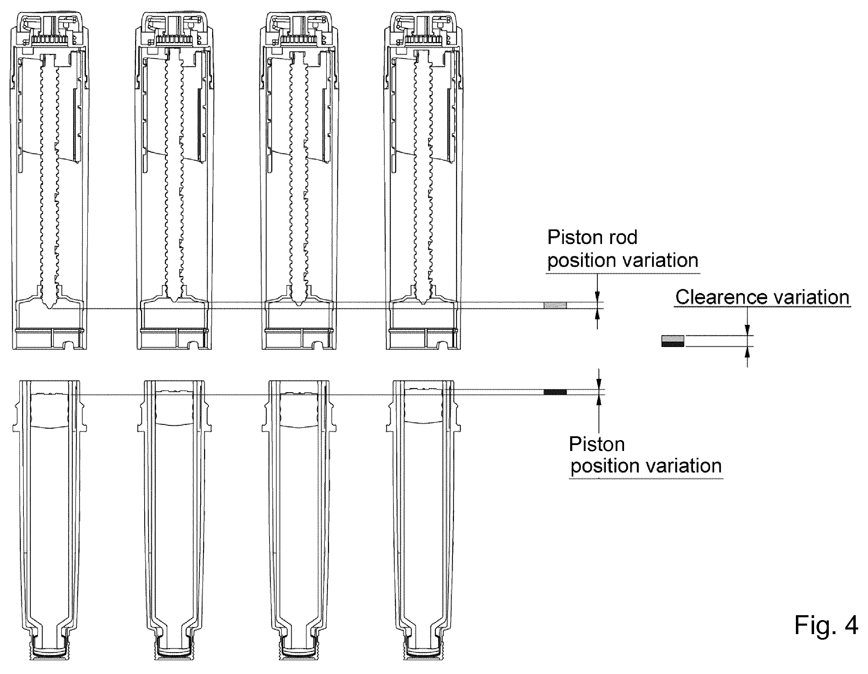

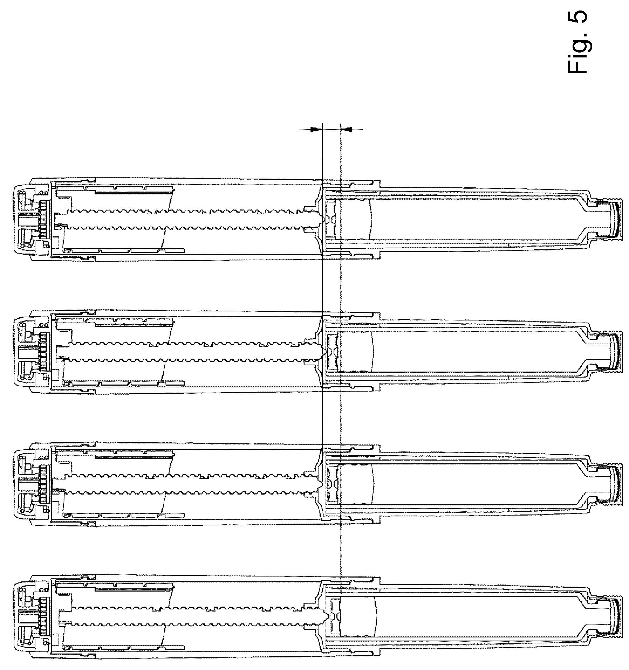

Production tolerances on the piston rod, the expelling mechanism, cartridge body, cartridge filling level and other components result in variations in piston rod position and piston position in each device during assembly. Such variation is schematically represented in figs. 4 and 5. In these figures, for reasons of clarity, only components serving understanding the problem associated with tolerance variations are shown whereas other components have been omitted from the drawings.

In exemplary embodiments, during assembly of the pen device, the cartridge may be initially inserted in the cartridge holder to form a first sub-assembly. The dose setting and expelling mechanism including the piston rod is mounted relative to the housing to form a second sub-assembly. Due to tolerances the position of the piston of the cartridge varies from one cartridge to another. To be able to compensate for tolerance variation, the position of the cartridge piston is therefore determined relative to a fixed reference point on the cartridge holder. Also the piston rod position in the housing can be determined relative to a fixed reference point on the housing.

Before the two sub-assemblies are finally mounted relative to each other a piston washer having a predefined standard geometry is brought into engagement with the piston of the cartridge. If no further compensation of the tolerance variations were made, the final pen assemblies would assume the states shown in fig. 5 where potentially axial clearance of varying magnitude would be present.

Hence, during assembly, and in order to minimize a potential axial clearance between the piston rod and the piston of the cartridge, positioning may be carried out by initially positioning the piston rod 220 in a nominal position. Due to tolerances various different clearance gaps between the piston and the piston rod will show when the distal and proximal subassemblies of each sample are permanently secured together. On the basis of measurements or estimations, which may be performed at different steps of the assembly process, the actual clearance gap in each sample may be eliminated or at least partly reduced by operating the dose setting and expelling mechanism. Automated operation of the dose setting and expelling mechanism may be carried out either after final assembly or prior to final coupling of the different subassemblies. However such compensation procedure means that the end-of-content mechanism will be operated to a lesser or higher degree even before the device is shipped to the user meaning that the experienced total doseable volume varies from sample to sample. Generally such variations and inconsistencies from one sample to another should be avoided as this may provide the impression that the quality of the device could be somewhat non-optimal.

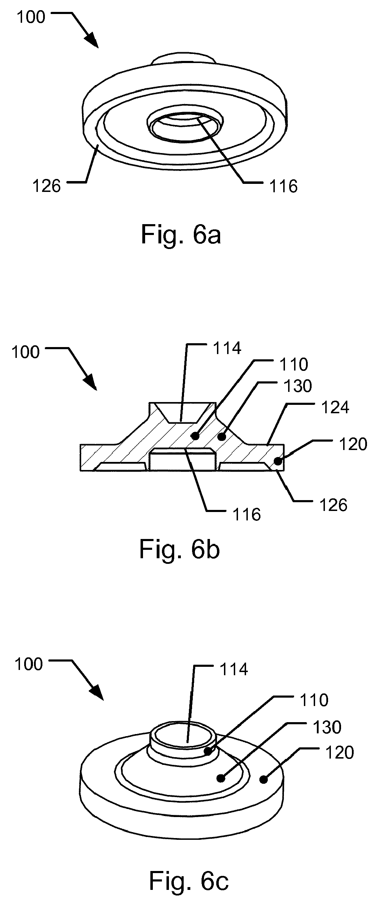

Turning to fig. 6a through fig. 8 an embodiment of an adjustable size piston washer 100 will be described. The washer 100 is intended for replacing the washer 227 of the prior art device described above seeking to eliminate or reduce the above problem of varying total doseable volume across different pen samples. However, this is only a non-limiting example and the washer as herein described may be used in conjunction with other injection devices, whether single shot or multi-shot injection devices.

The piston washer 100 serves in a drug delivery device to transfer a distally directed axial force from a piston rod towards a piston of a held cartridge. The piston washer 100 comprises a proximal surface geometry 114 adapted for engagement with the distal portion of the piston rod and a distal surface geometry 126 configured to engage and abut the piston of a held cartridge.

Figures 6a, 6b and 6c respectively show the piston washer 100 in a perspective distal view, a cross-sectional plan view, and a perspective proximal view. The piston washer 100 is in figs. 6a-6c shown in a state where the piston washer has a geometry which has been fine-tuned, by means of an adjustment process, relative to an initial state.

As shown in figs. 6a-6c the washer 100 is formed as a rotation symmetrical member which is to be arranged with its axis of symmetry along the expelling axis of a drug delivery device. The washer 100 comprises a central section 110, a peripheral section 120 and a bridge section 130 interconnecting the central section and the peripheral section.

At shown in the lower side of the drawing 6b, i.e. the distal part of the piston washer 100, the washer forms the peripheral section 120 shaped as a cylindrical flange which defines a distal peripheral surface 126 and a proximal peripheral surface 124. The central section 110 defines a distal central surface 116 and a proximal central surface 114.

The proximal surface of the piston washer 100 defines a recessed central area 114 configured for receiving and engaging the distal portion of a piston rod. In the shown embodiment, the piston washer 100 is arranged to be rotatably engaged with the piston rod so that the piston rod is rotatable relative to the piston washer exhibiting minimal friction against rotation. In the shown embodiment, the distal surface of the peripheral section 120 defines a planar annular peripheral ring. The distal surface of central section 110 defines a central recessed area 116 for receiving a tool part (to be described below). In the shown embodiment the bridge section 130 is shaped as an annular section which at its radially outwards facing surface axially tapers into a smaller diameter at its proximal end.

In the shown embodiment the piston washer 100 defines a proximal surface geometry 114 configured for engagement with the distal portion of a piston rod and a distal surface geometry 126 configured for engagement with the proximal surface of a piston of a cartridge. The proximal surface geometry 114 and the distal surface geometry 126 are arranged axially relative to each other with a target axial spacing. The target axial spacing which may be dimensioned so that, when arranged axially in a device between the piston rod and the piston, the piston washer 100 fills the axial gap between the piston rod and the piston and ensures full engagement between these components as the device becomes fully assembled.

In other embodiments, the target axial spacing may be dimensioned so that the piston rod and the piston washer cause a slight compression of predefined magnitude of the piston when the device becomes fully assembled, In still further embodiments, the target axial spacing may be dimensioned so that, with the device in the fully assembled state, a minor predefined air gap is introduced somewhere between the piston rod and piston so that a predefined axial movement of the piston rod is required before the piston is moved axially.

In accordance with an aspect of the invention, differently sized piston washers may be prepared by using a standard sized piston washer that in an initial state defines an initial axial spacing between the proximal surface geometry 114 and the distal surface geometry 126. In accordance with the present invention, the piston washer is prepared by subjecting the piston washer to a deformation process for modifying the initial axial spacing between the proximal surface geometry 114 and the distal surface geometry 126 into a target axial spacing. Hence, as various components designated for the individual pen samples are prepared and assembled during production, on the basis of determinations or estimations, such as by performing measurements, a target axial spacing for a particular piston washer designated for the individual pen sample may be determined. Thus, the piston washer may be prepared by deformation until it exhibits the target axial spacing. The preparation of the washer may thus occur as part of in-line production.

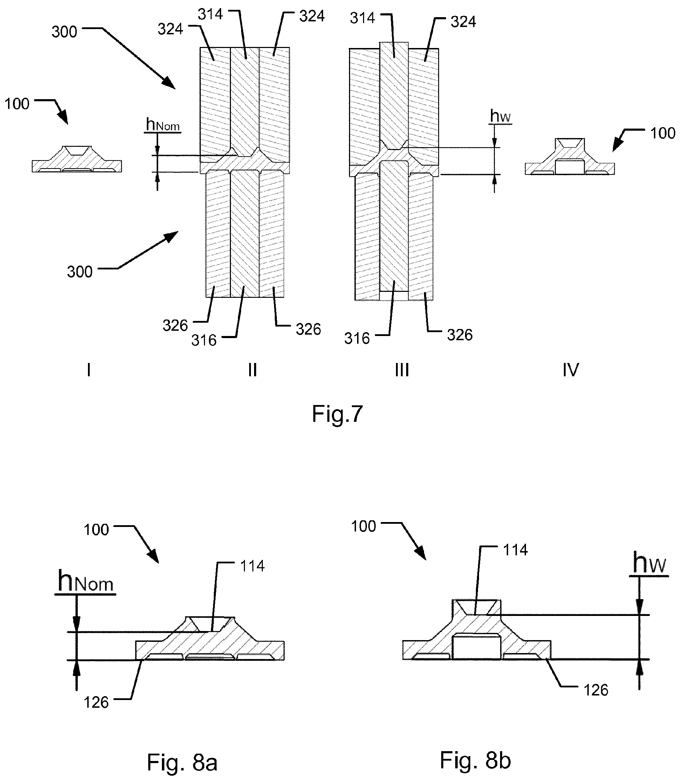

It is to be noted that above mentioned axial spacing generally may refer to an axial interface location between the piston rod and the proximal surface of the piston washer and an axial interface location between the distal surface of the piston washer and the piston, respectively. Thus, the axial spacing need not necessarily refer to the lowest point of the recessed central area 114 of the proximal central surface and the lowest point of the distal peripheral surface 126. Fig. 7 schematically shows various steps of an embodiment process for preparing the piston washer 100. In the shown embodiment, as shown in view I, a piston washer 100 is provided defining an initial axial spacing corresponding to a nominal height h_{Nom} between the proximal surface geometry 114 and the distal surface geometry 126. The piston washer may for example be provided by a moulding process. The piston washer is to be subjected to a press deformation process using a tool arrangement 300 comprising matched tools for engaging the various surface geometries of the piston washer.

In the shown embodiment, the tool arrangement 300 comprises first and second cylindrical fixtures for axially clamping the peripheral section 120 of the piston washer 100. Fig7, II shows a lower die 326 configured for engaging the distal peripheral surface 126 of the piston washer and an upper die 324 configured for engaging the proximal peripheral surface 124. A lower cylindrical mandrel 316 is configured for engaging the distal central surface 116 whereas an upper cylindrical mandrel 314 is configured for engaging the proximal central surface 114.

As shown in fig. 7, II, the piston washer 100 is arranged in the tool arrangement 300 so that the lower die 326 and upper die 324 axially clamps and retain the peripheral section 120 of the piston washer. The lower die 326 and upper die 324 also partly supports the bridge section 120. The lower cylindrical mandrel 316 has been brought into mating engagement with the distal central surface 116 whereas the upper cylindrical mandrel 314 has been brought into mating engagement with the proximal central surface 114.

Fig. 7, III shows the arrangement in a state wherein the lower cylindrical mandrel 316 and the upper cylindrical mandrel 314 has been moved axially in the proximal direction a given distance relative to the lower and upper dies 326/324. The movement causes the piston washer to plastically deform and assume the shape conforming to the position of the various tool parts. As shown, the piston washer now assumes a modified target axial spacing corresponding to the required washer height h_{W} between the proximal surface geometry 114 and the distal surface geometry 126.

By using two axially movable mandrels 316/314 the central section 110 is dragged in the proximal direction relative to the peripheral section 120. The movement of the mandrels 316/314 will typically occur simultaneously by a correlated movement in the proximal direction, such as by being moved in synchronism. However, in accordance with other embodiments, the mandrels 316/314 may be moved axially with different velocities and with different magnitudes.

In fig. 7, IV the piston washer 100 has been removed from the tool arrangement 300 and the figure thus show the piston washer in its final state ready for being inserted and assembled with the remaining parts of an injection device.

Fig. 8a and 8b provide enlarged views of the piston washer before and after deformation. As will be readily acknowledged, a series of piston washers 100, each assuming their initial state, are modifiable into a range of different washers having respective different target axial spacing to adapt to the range of possible tolerance variations of the particular injection devices they are designated for.

In one embodiment a piston washer in its initial state is adapted for modification such that the proximal surface geometry is moved in the proximal direction relative to the distal surface geometry in magnitude selected from a range of different magnitudes and in accordance with the required increase in axial spacing relative to the nominal height H_{Nom}. In other embodiments the initial axial spacing is so chosen that the piston washer exhibits a relatively large initial axial spacing and which is configured for being deformed by a reduction in height by a magnitude selected from a range of different magnitudes. In still other embodiments, the piston washers may be configured for a mid-level initial axial spacing and be deformed either for a reduction or an increase in axial spacing in accordance with the required axial spacing.

As will be acknowledge, the amount of deformation may be applied in steps or arbitrarily within a range of deformations. In other embodiments, instead of using a tool arrangement having movable mandrels, a series of different press stations providing different degrees of deformation may be arranged so that a specific one of the press stations is selected in accordance with the required axial spacing for a required piston washer. Alternatively, a series of different press stations may be successively used for gradually providing increased deformation to the individual piston washer.

Suitable non-limiting examples of materials useful for being plastically deformed and to be used for the piston washer or for being comprised within the piston washer may be chosen as Polypropylene (PP) or Polyoxymethylene plastic (POM). Other suitable materials may include a metal, such as aluminium or a metal alloy. In the shown embodiment, the entire piston washer is fully made of either PP or POM. In other embodiments the piston washer is only partly made of a plastically deformable material PP or POM. For example the bridge section 130 and part of the central section 110 may be made of a plastically deformable material whereas the peripheral section 120 may be made from a different material.

In accordance with the particular choice of material for the piston washer, the deformation of the piston washer will typically be carried out while applying heat to the piston washer.

Non-limiting examples for piston washers may include washers having a target axial spacing selected in the range of 1.5 mm to 5 mm, such as between 2 mm to 4 mm, such as between 2.5 to 3.5 mm.

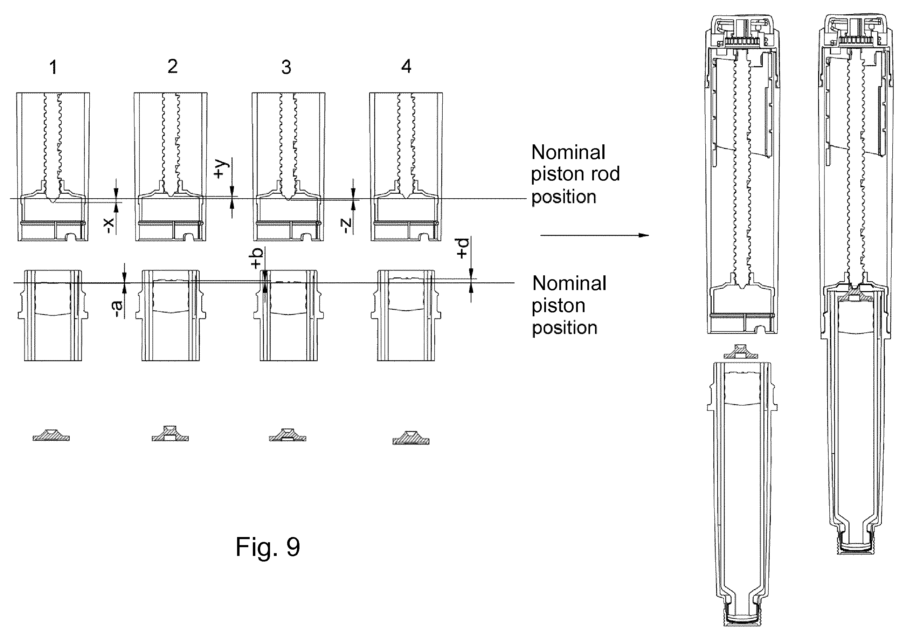

Fig. 9 schematically shows key components for four different pen samples, where each pen sample exhibit tolerance variations both with respect to the nominal piston position in the first sub-assembly and with respect to the nominal piston rod position of the second sub-assembly. For each first sub-assembly and second sub-assembly designated for subsequently mating and coupling, in accordance with determined or measured tolerance variations for the sub-assemblies, a designated piston washer is prepared by the above described deformation process to eliminate, or at least partly compensate, for the axial clearance between the piston rod and the piston.

As shown in the right hand side of fig. 9, the piston washer is subsequently arranged between the piston rod and the piston thereby partly or completely filling the axial clearance between these components. As such, operation of the dose setting and expelling mechanism is not required during assembly operations of the pen devices. However, if desired, for example for performing a function test of the dose setting and expelling mechanisms, a dose setting and expelling operation of a standardized magnitude may be provided. This may be compensated for when adjusting the individual piston washers. In accordance herewith, the total dosage volume for each pen device can be made constant across individual pen samples.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the true spirit of the invention.

## Claims

1. A method of preparing a piston washer for use in a drug delivery device and for transferring a distally directed axial force from a piston rod towards a piston of a held cartridge, comprising the steps of:
a) providing a piston washer comprising a deformable portion made from a plastically deformable material, the piston washer defining a proximal surface geometry configured for engagement with the distal portion of a piston rod and a distal surface geometry configured for engagement with the proximal surface of a piston of a cartridge wherein the proximal surface geometry and the distal surface geometry are arranged axially relative to each other with an initial axial spacing,
b) arranging the piston washer in a tool arrangement,
c) using the tool arrangement to apply plastical deformation of the deformable portion of the piston washer thereby modifying the axial spacing between the proximal surface geometry and the distal surface geometry until a target axial spacing is obtained.

2. A method of preparing a piston washer as defined in claim 1,
wherein the piston washer defines a peripheral section and a central section, wherein the peripheral section comprises a distal peripheral surface and a proximal peripheral surface and wherein the central section defines a distal central surface and a proximal central surface, and wherein the proximal central surface includes a recessed central area configured for receiving the distal portion of the piston rod.

3. A method of preparing a piston washer as defined in claim 2, wherein an extreme distal surface of the piston washer is defined by the distal surface of the peripheral section.

4. A method of preparing a piston washer as defined in any of the claims 2-3, wherein the piston washer includes a bridge section interconnecting the central section and the peripheral section and wherein the bridge section at least partly defines said deformable portion.

5. A method of preparing a piston washer as defined in any of the claims 2-4, wherein the method step c) of applying plastical deformation includes the step of applying controlled pressure on the distal central surface and/or the proximal central surface for axially moving the central section of the piston washer relative to the peripheral section.

6. A method of preparing a piston washer as defined in any of the claims 2-5, wherein the tool arrangement comprises a first peripheral tool portion configured for engaging the distal peripheral surface of the piston washer and a second peripheral tool portion configured for engaging the proximal peripheral surface of the piston washer and wherein the method step b) of arranging the piston washer in a tool arrangement comprises the step of axially clamping the peripheral section of the piston washer between the first peripheral tool portion and the second peripheral tool portion.

7. A method of preparing a piston washer as defined in claim 6, wherein a first central tool portion is arranged for engaging the distal central surface of the piston washer and a second central tool portion is arranged for engaging the proximal central surface of the piston washer, and wherein the step of applying plastical deformation is provided by moving the first central tool portion and/or the second central tool portion axially relative to the first and second peripheral tool portion.

8. A method of preparing a piston washer as defined in the claim 7, wherein the first central tool portion and the second central tool portion axially engage the central section of the piston washer on respective sides and wherein the first central tool portion and the second central tool portion are moved axially in the same direction relative to the first peripheral tool portion and the second peripheral tool portion to reduce or increase the axial spacing between the proximal surface geometry and the distal surface geometry.

9. A method of preparing a piston washer as defined in claim 8, wherein the first central tool portion and the second central tool portion are moved axially in synchronism relative to the first peripheral tool portion and the second peripheral tool portion to reduce or increase the axial spacing between the proximal surface geometry and the distal surface geometry.

10. A method of preparing a piston washer as defined in any of the claims 1-9, wherein the deformable portion of the piston washer is heated prior to or during plastical deformation of the deformable portion.

11. A piston washer for use in a drug delivery device for transferring a distally directed axial force from a piston rod towards a piston of a held cartridge, the piston washer comprising:
- a proximal surface geometry configured for engagement with the distal portion of a piston rod, and
- a distal surface geometry configured to engage and abut a piston of a held cartridge,
wherein the piston washer comprises a deformable portion made from a plastically deformable material and wherein the proximal surface geometry and the distal surface geometry are arranged axially relative to each other with a target axial spacing obtained by deformation of the deformable portion

12. A method of assembling a drug delivery device comprising a piston washer as prepared by the method as defined in any of the claims 1-10, the method comprising the steps of:
- providing a cartridge comprising a liquid drug and a piston slideably arranged therein in a first housing component to form a cartridge sub-assembly,
- providing a dose setting and expelling mechanism comprising the piston rod in a second housing component to form a housing sub-assembly,
- determining an axial position of a proximal end face of the piston of a cartridge of the cartridge sub-assembly,
- determining an axial position of a distal end face of a piston rod of a drive mechanism pre-assembled in a second housing component,
- determining or estimating the size of axial clearance between the piston and the piston rod if the first housing component and the second housing component were assembled,
- providing the piston washer by the method of preparing a piston washer as defined in any of the claims 1-10, wherein the target axial spacing corresponds to or correlates with said axial clearance, and
- mutually interconnecting the first and the second housing components with the piston washer positioned between the piston rod and the piston.

13. A drug delivery device for expelling a dose of drug from a held cartridge, the drug delivery device defining a distal drug outlet end and an opposite proximal end and comprising:
- a housing component holding a cartridge comprising a liquid drug and a piston slideable arranged therein in an axial direction,
- a dose setting and expelling mechanism coupled to the housing component and comprising a piston rod for exerting a force on the piston of the cartridge in a distal direction for expelling a dose, and
wherein a piston washer prepared according to any of the claims 1-10 is arranged axially between the piston of the cartridge and the piston rod.

14. A drug delivery device as defined in claim 13, wherein the piston rod is configured for rotation during dose expelling and wherein the piston rod is rotatably arranged relative to the piston washer to allow the piston rod to rotate while the piston washer remains non-rotatable.
